(19) **Europäisches Patentamt**
**European Patent Office**
**Office européen des brevets**

(11) **EP 1 907 830 B1**

(12) **EUROPEAN PATENT SPECIFICATION**

(45) Date of publication and mention
of the grant of the patent:
**30.09.2020 Bulletin 2020/40**

(51) Int Cl.:
***G01N 21/89*** *(2006.01)*      ***D01H 13/22*** *(2006.01)*
***G01N 33/36*** *(2006.01)*

(21) Application number: **05793769.0**

(22) Date of filing: **13.10.2005**

(86) International application number:
**PCT/IB2005/003167**

(87) International publication number:
**WO 2007/010325 (25.01.2007 Gazette 2007/04)**

(54) **DETECTING FOREIGN SUBSTANCES IN A TEXTILE MATERIAL**

NACHWEIS VON FREMDSTOFFEN IN EINEM TEXTILMATERIAL

DÉTECTION DE SUBSTANCES ÉTRANGÈRES AU SEIN D UN MATÉRIAU TEXTILE

(84) Designated Contracting States:
**CH DE IT LI TR**

(30) Priority: **22.07.2005 IN CH09802005**

(43) Date of publication of application:
**09.04.2008 Bulletin 2008/15**

(73) Proprietor: **Premier Evolvics PVT. Ltd.**
**Coimbatore 641 005 (IN)**

(72) Inventors:
• **RAMACHANDRAN, Shekaripuram,**
**Narayanaswamy**
**Coimbatore 641 038 (IN)**

• **SUNDARAM, Balasubramaniam, Shamuga**
**Podanur,**
**Coimbatore 641 023 (IN)**
• **KAVITHA, Narayanaswamy**
**Podanur,**
**Coimbatore 641 023 (IN)**

(74) Representative: **Frei Patent Attorneys**
**Frei Patentanwaltsbüro AG**
**Postfach**
**8032 Zürich (CH)**

(56) References cited:
**EP-A- 0 399 945      EP-A1- 0 652 432**
**WO-A-93/13407      DE-U1- 29 719 245**

**Description**

**FIELD OF THE INVENTION**

The invention relates to an apparatus and a method for detecting foreign substances in a textile material, in particular for detecting the presence of contaminants in a strand-like textile material such as a yarn, as described in the preamble of the corresponding independent claims.

**BACKGROUND OF THE INVENTION**

US 6,175,408 describes a measurement apparatus emitting white light and measuring the intensity of light reflected from a travelling yarn in the red, green and blue frequency bands. For each of the three possible pairs of intensity signals a difference signal is formed. A contamination is deemed to be detected when at least one of the difference signals exceeds a given threshold. The focus of the invention is on the use of a frequency transformer for generating white light over a wider frequency spectrum, avoiding the use of narrow-band, single colour LED's.

EP 0 399 945 B1 also shows an apparatus with a polychromatic light source in which the difference between the received intensities for two colours is used to detect a contaminant. In an alternative embodiment, light is emitted at two wavelengths and detected by a broadband detector, as in the following reference:.

WO 03/008950 shows a device emitting light at two different wavelengths, and having a single light receiver. Since the dependency of reflection on wavelength differs according to material, the amount of total reflected light depends on the presence and the kind of contaminant material. In this manner, it should be possible to distinguish between different contaminants.

US 5,915,279 shows a measurement setup for detecting parameters of an object in general terms. A polychromatic light source emitting light at at least two different wavelengths, and a plurality of detectors for different colour bands are used. Methods for the statistical analysis of a plurality of parameters are described, e.g. light intensities detected at different wavelengths, and including time histories of intensities in order to determine deviations. However, the resulting algorithms are complicated and too general to be immediately applicable.

EP 0 652 432 A1 discloses the use of sensors for two wavelengths, with the optical arrangement ensuring that the light received by the sensors comes from exactly the same location of the yarn. The light received may lie in the infrared or near-infrared spectrum. From the detector signals corresponding to received intensities, a signal corresponding to a quotient (i.e. a difference of logarithms) is formed, in order to eliminate the influence of yarn diameter, structure etc.

WO 93/13407 discloses the detection of contaminants using two light sources having non-overlapping spectra, e.g. one in the near infrared range, and two corresponding sensors. A moving yarn is observed, and the sum of changes in reflected and transmitted light (after filtering) is used to indicate the presence of a contaminant.

**DESCRIPTION OF THE INVENTION**

It is an object of the invention to create an apparatus and a method for detecting foreign substances in a textile material of the type mentioned initially, which is simple to implement and improves the detection quality.

These objects are achieved by an apparatus and a method for detecting foreign substances in a textile material according to the corresponding independent claims.

The apparatus for detecting foreign substances in a textile material, in particular a strand-like textile material such as a yarn, comprises a first light source for emitting visible (VIS) light, a second light source for emitting radiation in the infrared (IR) spectrum, a first detector sensitive to visible light reflected from the textile material and configured to generate a corresponding first detector signal, and a second detector sensitive to radiation in the IR spectrum reflected from the textile material and configured to generate a corresponding second detector signal. The first and second light sources and first and second detectors are arranged to illuminate and view the same section of the textile material, and a signal processing section is configured to determine a weighted difference of the two detector signals and outputting a signal indicative of the presence of a contaminant whenever said difference exceeds a threshold value. "Weighted difference" means that at least one of the detector signals is multiplied by a substantially constant factor before the difference is determined.

The second light source emits light in the near-visible range as an infrared (IR) light source.

The invention may be applied to an elongated, strand-like textile material such as a yarn or roving or sliver, but also to chunks of cotton being conveyed or blown past the detecting arrangement. In general, contaminants as well as the textile material reflect light both in the Visible and the Infrared Spectrum. However, the proportion of reflected light in the two spectra varies between a yarn and a contaminant. For example, with a black yarn as a contaminant, the VIS detectors will have less reflected light in the Visible Spectrum but the Infrared detectors will still observe reflected light roughly the same as for the normal yarn. Variations in the yarn diameter, hairiness, position etc. affect both the spectrums proportionally.

The reflection of the yarn in the visible (VIS) and the infrared (IR) spectrum are recorded from a normal running yarn. The two recorded signals may be combined to form a ratio or spectral ratio for the normal running yarn by a long term averaging process. In a preferred embodiment of the invention, this long term average of this spectral ratio for the normal running yarn is normalized to unity. Variations in yarn diameter and hairiness of a yarn to be tested affect both the reflected visible (VIS) and the infrared (IR) spectrum in a similar manner. Typical contaminants distinctly change the reflection behaviour in the visible (VIS) and infrared (IR) spectrum compared to that of the reference yarn, i.e. uncontaminated yarn material.

In another preferred embodiment of the invention, due to the differences in light intensity, sensor efficiency and reflectivity in the two bands of radiation, the two detected signals are weighted with respect to one another, such that their weighted difference becomes zero for the normal running yarn. A weighting coefficient can be adapted in accordance with long-term averages of the first and second detector signal. In a preferred embodiment of the invention, the weighted difference Dw is normalised with respect to the second detector signal, e.g. by dividing it by the IR detector signal.

In a further preferred embodiment of the invention, the first and second detector are arranged to observe, i.e. to measure radiation from a first angle with respect to the yarn, and the device comprises a further VIS and a further IR detector arranged to observe, from a second angle with respect to the yarn. The first and second angle show their maximum value when projected onto a plane that is perpendicular to the running yarn. The first and second detectors constitute a first detector set, and the further detectors constitute a second detector set. The further detectors are sensitive to the same wavelengths as the first and second detector, respectively.

In yet a further preferred embodiment of invention, a third detector is placed opposite to the light sources, to detect the positional variations of the yarn. The third detector is sensitive in either the VIS or the IR or both frequency ranges. Preferably, the position detectors generate detector signals that are used in classifying situations where the reflected signals alone may not take into account certain disturbances. For example, the spectral ratio or the weighted difference Dw may also change when a position variation of the yarn occurs. Taking the third detector signal into account, a position variation can be distinguished from a contaminant signal.

Preferably, the position detectors generate detector signals that are used in classifying situations where the reflected signals alone may not take into account certain disturbances.

In a further preferred embodiment of the invention, the first and second light source are identical, that is, a single source or a combined source emits both the visible and the IR light used for the measurements.

The method for detecting foreign substances in a strand-like textile material, comprises the steps of claim 9.

The respective deviations are preferably determined with respect to constant reference values or long term reference values.

In particular, this may done according to the following method:

- Determine the ratio of the two spectral signals. Compare the instantaneous signal value with a minimum threshold limit which is a small percentage above than of the yarn average. If the signal exceeds this limit for a predetermined length, then qualify this as being a contaminant.
- Compute the deviation in the spectral signal in the visible spectrum with respect to the reference material i.e. yarn. This deviation allows to identify a first class of contaminants such as Hair, Seed Coats etc.
- Compute the deviation in the spectral signal in the IR spectrum with respect to the reference material. This deviation allows to identify a second class of contaminants such as Polypropylene (PP).
- Compute the ratio of the two deviations and use this ratio to further categorise the contaminant as being one of specific materials like Hair, Seed Coats, PP, Jute, Coloured Yarn etc.

Further preferred embodiments are evident from the dependent patent claims.

**BRIEF DESCRIPTION OF THE DRAWINGS**

The subject matter of the invention will be explained in more detail in the following text with reference to preferred exemplary embodiments, which are illustrated in the attached drawings, in which:

Figure 1    schematically shows a first testing arrangement;
Figure 2    schematically shows an arrangement of sources and detectors;
Figure 3    schematically shows another arrangement of sources and detectors;
Figure 4    schematically shows a testing arrangement using a position sensor;
Figure 5    shows an arrangement for observing a flow of cotton chunks;
Figure 6    shows an exemplary trajectory of signals representing reflected visible and IR light;
Figure 7    shows an exemplary trajectory of a weighted difference signal; and

Figure 8      illustrates a classification method according to a preferred embodiment of the invention.

The reference symbols used in the drawings, and their meanings, are listed in summary form in the list of reference symbols. In principle, identical parts are provided with the same reference symbols in the figures.

## DETAILED DESCRIPTION OF PREFERRED EMBODIMENTS

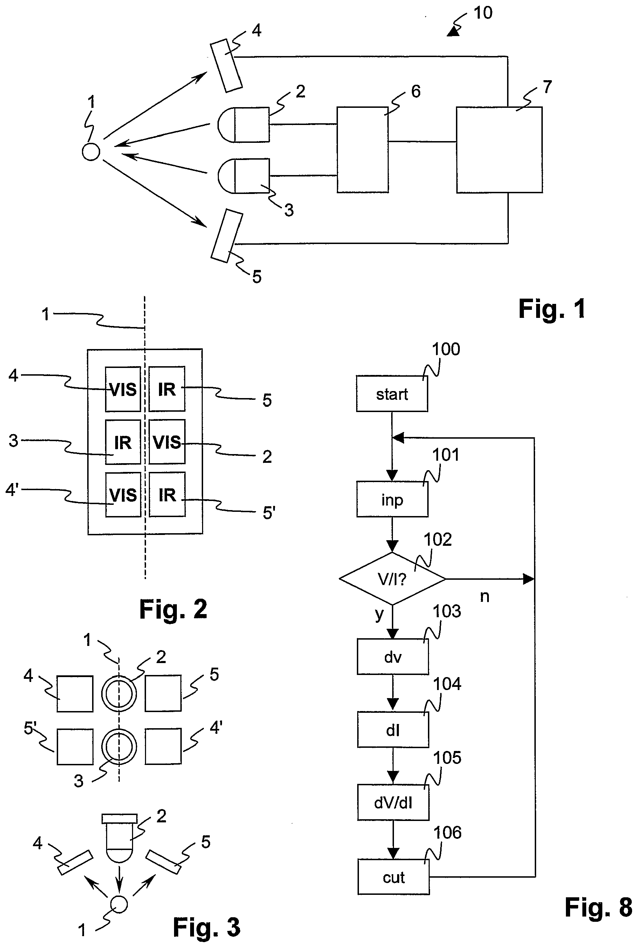

**Figure 1** schematically shows a first testing arrangement with a yarn 1 seen head-on, a first source 2 and second source 3 arranged to illuminate the same section of the yarn 1 and a first detector 4 and second detector 5 arranged to receive light reflected from said section of the yarn 1. The first source 2 and second source 3 are powered by a driving circuit 6. The signals from the first detector 4 and second detector 5 are processed by a signal processing section 7 by means of analog or digital signal processing elements. The first detector 4 and second detector 5 constitute a first detector pair 10 arranged to observe a first side of the yarn 1.

**Figure 1** is highly schematic in that it only shows the functional relation between the elements, but not the true geometric relations. **Figure 2** shows an arrangement of sources and detectors for observing the yarn 1. The sources and detectors are arranged in two rows along the length of the yarn 1, with the source for each part of the spectrum (IR, VIS) observed lying in one row, and two corresponding detectors for that part of the spectrum (IR, VIS) lying in the other row. For example, the first source 2, emitting in the visible spectrum, lies in the right row, and the visible light (VIS) detector 4 and a further VIS detector 4' lie in the left row. The second source 3 emitting in the infrared (IR) spectrum and the IR detector 5 and a further IR detector 5' are arranged vice versa. In a preferred embodiment of the invention, the signals of the VIS detector 4 and the further VIS detector 4' are added to provide the VIS detector signal for further processing, and the IR detector 5 signal and further IR detector 5' are added to give the IR detector signal for further processing. In another preferred embodiment the various signals of the VIS and the IR Detectors 4, 4',5, 5' could be processed independently.

For practical reasons, since VIS detectors usually are sensitive to IR light as well, the VIS detectors used here include an IR blocking filter. The arrangement according to Figure 2 will observe a major part of one side of the yarn 1, e.g. ca. 135° of the yarn's circumference, which is sufficient to detect contaminants wound around the yarn 1.

**Figure 3** shows another preferred arrangement of sources and detectors for observing the yarn 1. The arrangement is shown in a view perpendicular to the yarn 1 and a roughly planar arrangement of sources and detectors (top), and in a view in the direction of the yarn 1 (bottom). The sources and detectors are arranged in three rows along the length of the yarn 1, the first row comprising a VIS detector 4 and a further IR detector 5', the second row comprising the first source 2 and the second source 3, and the third row comprising an IR detector 5 and a further VIS detector 4'. Having two detector pairs 4, 5'; 5, 4' lying essentially in a plane along the yarn 1 and observing the yarn 1 from different sides gives a good coverage of the test material under consideration. The signals from the second pair are independently processed by means of analog or digital processing elements.

In the signal analysis for the two pairs of sensors, the signals of each pair of IR/VIS sensors may be processed as shown below, and the output signals detecting the presence of a contaminant may be AND / OR combined. Alternatively, the signals of the two visible light detectors 4, 4' are summed and the signals of the two IR detectors 5, 5' are summed, and the summed signals are processed as shown below.

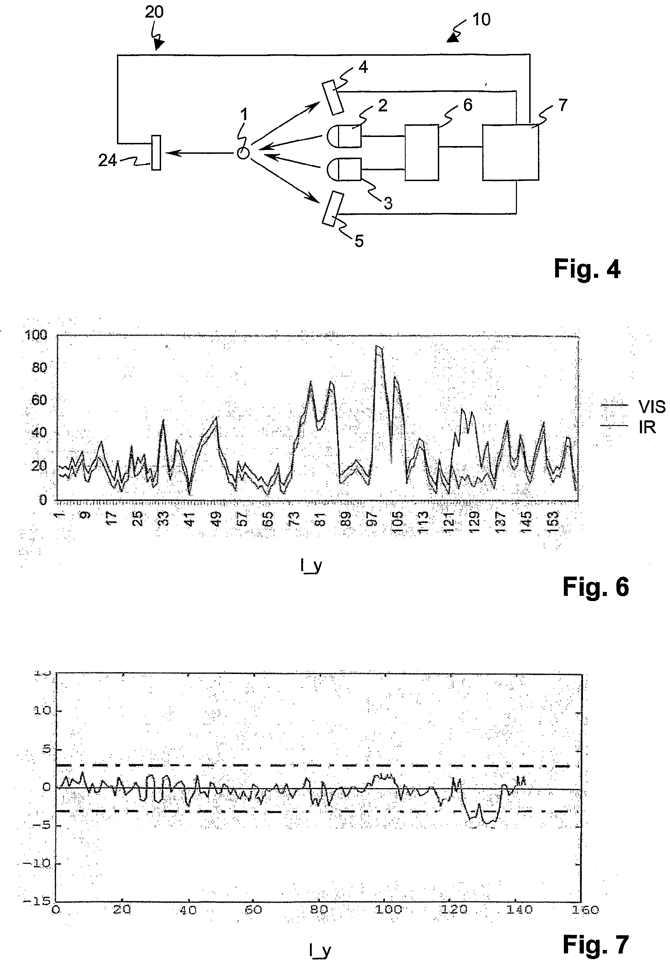

**Figure 4** schematically shows a second testing arrangement, further comprising a third detector 24 operating as a position detector, and whose detector signals are e.g. processed by the signal processing section 7 as well. The position detector is arranged, with respect to the yarn 1, in opposition to the respective source, such that the light emitted by the source casts a shadow of the yarn 1 on the detector. The position detector 24 is sensitive to the visible and/or IR light.

Depending on the position of the yarn in the detecting apparatus, the position sensor signal will vary. Similar variations may also be observed due to changes in thickness or hairiness of the yarn (and can be used to determine thickness and/or hairiness). Therefore, the actual state and position of the yarn is inferred from a combination of the position signal and the reflected signals in the visible and IR spectrum. This inference or discrimination of different states is based on the following principles:

- With no yarn 1 present, the position detector 24 receives the maximum light intensity, denoted in the following with the position signal value 100.
- When the yarn 1 is placed inside the detector slot in its nominal position, then the yarn 1 obstructs the light falling on the detector 24 and hence less light is received. The position sensor value lies around 100-k, with (k>0). The reflected signals in the visible and IR spectrum show their nominal or reference values, denoted b and c, respectively.
- A thicker yarn 1 results in even less light received, and a thinner yarn in more light received by the position detector 24, corresponding to position signal values of 100-k' and 100-k", respectively, where e.g. k'=2k and k"=k/2. The reflected signals increase for a thicker and decrease for a thinner yarn, e.g. to double or half their nominal value. Depending on actual circumstances, another factor than 2 may be chosen to define a thick or thin yarn.

- When yarn 1 moves closer to the position detector 24, the shadow cast becomes thinner, e.g. with a position signal value 100-j, with (j< k). The reflected signals decrease, e.g. to values b' and c', respectively, with (b'<b) and (c'<c).
- When then yarn 1 moves towards the source, away from the position detector 24, more light is blocked, and the position signal becomes e.g. 100-1, with (1>k). The reflected signals increase, e.g. to values b" and c", respectively, with (b">b) and (c">c).
- When the yarn moves outside the slot sideways from its intended position, it blocks light of lesser intensity. Hence the position signal becomes e.g. 100-m with (m < k). Due to the asymmetric arrangement of the visible and IR detectors, the reflected signal of one of them shall increase, and the other one decrease, e.g. to values around 2b for the visible and c/2 for the IR detector. Again, another factor than 2 may be appropriate. Eventually, when the yarn 1 leaves the detection zone, the position signal becomes 100, corresponding to a no yarn situation.
- The same holds for the position signal when the yarn 1 moves sideways into the slot, with a reference value 100-n with (n<k). However, the increase and decrease of the visible and IR reflected signals is the inverse from the above case. That is, the visible signal is around b/2 and the IR signal is around 2c. Again, another factor than 2 may be appropriate.
- A contamination spirally wound around the yarn will not change the position signal. The reflectance in the visible range shall be around a value x and in the IR range around a value y.

The actual values for the above constants b, b', b", c, c', c", i, j, k, l, m, n, x, y are determined experimentally according to the exact geometry of the detector arrangement and the properties of the yarn and contaminants.

The following table gives an overview of the different states that are distinguished by the different combinations of sensor values mentioned above. The second, third and fourth column give reference values for position, visible and IR sensor values corresponding to the respective state.

| State | Position | Reflectance 1 | Reflectance 2 | Description |
| --- | --- | --- | --- | --- |
| No Yarn | 100 | 0 | 0 | No Yarn in the Slot |
| Yarn | 100-k | b | c | Yarn is available in its intended position |
| Double Yarn | 100-2k | 2b | 2c | Yarn Diameter Doubles - Thick Place |
| Half Yarn | 100-k/2 | b/2 | c/2 | Yarn Diameter Decreases by Half - Thin Place |
| Y → S | 100-j | b' | c' | Yarn Moves near to the Position Detector |
| Y → R | 100-l | b" | c" | Yarn Moves near to the Reflectance Detectors |
| Y ^ | 100-m | 2b | c/2 | Yarn Moves Outside the Slot |
| Y v | 100-n | b/2 | 2c | Yarn Moves Inside the Slot |
| Contamination | 100-k | x | y | Contamination |

In a preferred embodiment of the invention, an electronic circuit or computer program distinguishes these cases by checking whether the measured values for each of the sensor are within a predetermined tolerance around the respective reference value. This checking can be done sequentially or in parallel or through a decision tree etc.

In a preferred embodiment of the invention, the first source 2 emits visible light and the second source 3 emits infrared light. In another apparatus, the second source 3 emits ultraviolet light. Thus, in both cases, the light emitted by the second source 3 is in the near-visible spectrum.

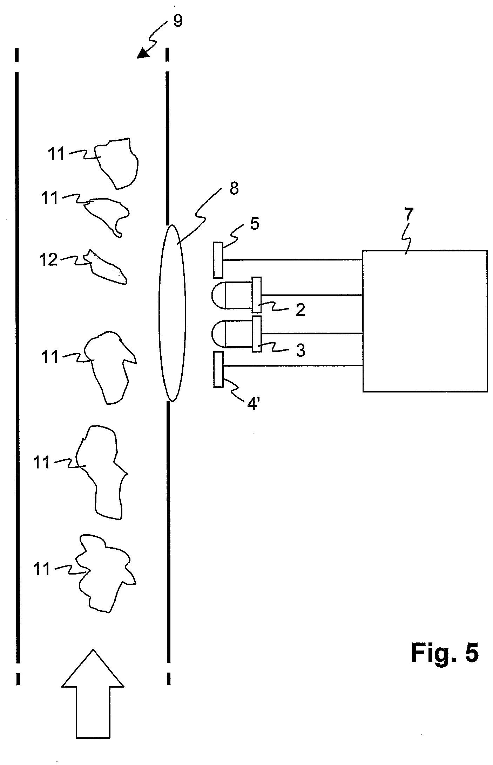

**Figure 5** shows another embodiment of the invention wherein the same arrangement of sources 2, 3 and detectors 4, 4', 5, 5' is utilized to detect contaminations 12 in chunks of cotton 11. Such arrangements are normally on the preparatory stages of the spinning mills. The detecting setup views the flow of the material that is blown through a material flow duct 9. The flow is viewed via a window, optionally incorporating a lens 8, which lets pass both the visible and infrared light. An array of the sensor arrangement 4, 4', 5, 5' is placed to cover the entire field of view, i.e. the entire cross section of the material flow duct 9. The signal processing is similar to detecting contaminants in a moving thread, but here serves to discriminate contaminant material 12 from chunks of cotton.

The following figures illustrate the characteristics and the use of detecting visible and infrared light. **Figure 6** shows an exemplary trajectory of signals representing reflected visible and IR light along the length l_y of the yarn being inspected. It can be seen that most of the time, or for most of the length of the inspected yarn 1, the two detector signals move in parallel. This movement corresponds to e.g. thickness, density, fluctuation, fibre distribution and hairiness variations. Between length 127 and 136 the detected visible light, with respect to the detected infrared light, drops of significantly.

**Figure 7** shows an exemplary trajectory of a weighted difference of the two signals. The weighted difference Dw, also called yarn deviation, is normalised with respect to the second detector signal, and is preferably computed according to

$$Dw = ( (k * Sv) - Si ) / Si$$

where Sv is the value of the first detector signal - for example, from the visible light detector - and Si is the value of the second detector signal - for example, from the IR detector - and k is a substantially constant weighting coefficient.

"Substantially constant" means that the value of k remains essentially the same and changes only slowly with regard to the values of the detector signals, e.g. with a time constant that is more than a hundred or a thousand times larger than the time constants of the changes in the detector signals. The weighting coefficient k can be adapted in accordance with a long-term average LTA of the first and second detector signal, e.g. as

$$k = LTA( Si ) / LTA ( Sv ) .$$

Where the LTA is the average computed over a time window of e.g. several seconds or minutes or tens of minutes, or over a length of several meters of running yarn.

Alternatively, the yarn deviation may be computed by computing the ratio Rs = Sv/Si and from this the yarn deviation Dw = (Rs - Ra)/Ra, with Ra = 1/k or the long term average of Sv/Si, that is, Ra = LTA(Sv/Si).

As seen in **Figure 7**, the weighted difference or yarn deviation remains within a certain band, and for the position (between length 127 and 136) in which a substantial difference between the two signal occurs, the weighted difference exceeds said band, indicating the presence of a contamination. For example, the predetermined threshold for detecting a contamination may be set to a small percentage value (a percent value of k as defined above is obtained by multiplying k with 100).

When illuminating the yarn 1 with the second source 3 (and fourth source 23) being ultraviolet emitters, with wavelengths e.g. from 254nm to 395nm, then certain contaminants such as polypropylene will fluoresce and convert UV light to visible light.

The position sensor signals are used as follows, e.g. in cases in which the weighted difference alone does not discriminate among different situations. The position signals

- may be the detector signals themselves, which are approximately proportional to the amount of light received by the detector,
- or they may be complementary signals, which are approximately proportional to the size of the shadow of the yarn 1 on the detector surface,

on the respective VIS /IR position detector 24.

The signal of the position detector 24 alone is used, in a preferred embodiment of the invention, to distinguish contaminations from vibrating yarn conditions. If the weighted difference indicates a contamination, which may also be caused by other disturbances, e.g. by the yarn vibrating, then the position signal is examined. If its change exceeds a predetermined threshold, then the situation is classified as being a vibration of the yarn instead of a contamination. Otherwise, a contamination is indicated and the corresponding section of the yarn 1 is cut out.

For detecting a change in the third detector signal, the position signal is e.g. compared to a substantially constant reference value such as a long term average. The flowchart of **Figure 8** illustrates a classification method according to a further preferred variant of the invention. In the initialisation step 100, labelled "start", processing begins. It is assumed that prior to this, a long term average reference value for the type of yarn being examined has been determined both in the visible (Yv) and in the infrared spectrum (Yi). From this, a reference ratio Y = Yv/Yi is computed. Alternatively an average of the ratios Y = LTA (Sv/Si) is computed.

In the input step 101, labelled "inp", a simultaneous sampling of the outputs of the VIS and IR detectors is performed. The observed signals are Sv and Si.

For the first comparison step 102, labelled "V/I?", the ratio S = Sv/Si is computed and compared to the reference ratio Y. If the absolute value of the deviation Dr = (Y-S)/Y exceeds a predetermined first threshold value, then the presence of a probable contaminant is indicated, and the procedure continues to categorise the contaminants by means of further steps, beginning with the second computation step 103. Otherwise, no contamination is indicated, and a further set of samples is processed in step 101.

For the second computation step 103, labelled "dV", a deviation Dv of Sv from Yv is computed. Preferably, Dv is normalised with respect to Yv, such that $Dv = (Yv-Sv)/Yv$. If the absolute value of the deviation Dv exceeds a predetermined second threshold value, then the presence of a certain probable types of contaminants, e.g. hair or seed coats, is confirmed, and the procedure continues to verify this by means of further steps, beginning with the third computation step 104.

For the third computation step 104, labelled "dI", a deviation Di of Si from Yi is computed. Preferably, Di is normalised with respect to Yi, such that $Di = (Yi-Si)/Yi$. If the absolute value of the deviation Di exceeds a predetermined third threshold value, then the presence of another set of contaminants such as Polypropylene is confirmed, and the procedure continues to verify this by means of the fourth computation step 105.

For the fourth computation step 105, labelled "dV/dI", the ratio Dv/Di is computed. This value is compared with predetermined spectral behaviour of different materials, i.e. yarn and contaminant materials, and contaminants are categorised accordingly.

If the Dr values indicating the presence of a specific contaminant exceed a predetermined clearing first threshold limit, the contaminant is to be cut from the yarn. This is done in step 106 labelled "cut", and a further set of samples is processed from step 101. A qualified contaminant is categorised and cut from the yarn.

Threshold values depend on the actual reference yarn and contaminant materials, and also on the type of sensor used and the spectral sensitivity curves of the sensors. For example, the following table gives measured values for the intensity of reflected light in the visible an IR spectrum for different materials.

| Spectrum | contaminant | | | | Reference |
|---|---|---|---|---|---|
| | PP | Hair | Jute | Black Yarn | White Yarn |
| VIS | 300 | 10 | 275 | 115 | 700 |
| IR | 750 | 120 | 1100 | 1630 | 1650 |
| IR / VIS | 2.50 | 12.00 | 4.00 | 14.17 | 2.36 |

The values observed in each spectrum in the contaminated yarn may be estimated analytically as being a weighted sum of the values from the contaminant and from the reference, the weight of the contaminant being typically between 15% and 50%. The other characteristic values or ratios described in the above are computed accordingly. Given these characteristic values and their stochastic distribution, the thresholds for distinguishing between different classes of contaminants and individual contaminants can be determined.

While the invention has been described in present preferred embodiments of the invention, it is distinctly understood that the invention is not limited thereto, but may be otherwise variously embodied and practised within the scope of the claims.

## LIST OF DESIGNATIONS

1       yarn
2       first source
3       second source
4, 4'   first detector
5, 5'   second detector
6       driving circuit
7       signal processing section
8       lens
9       material flow duct
10      first detector pair
11      cotton chunks
12      contamination
24      third detector, position detector

## Claims

1.  An apparatus for detecting foreign substances in a textile material, in particular a strand-like material such as a yarn (1), or a flow of pieces of textile material (11), comprising a first light source (2) for emitting visible light, a second (3) light source for emitting radiation in the infrared spectrum, a first detector (4) sensitive to visible light reflected from the textile material and configured to generate a corresponding first detector signal, and a second detector (5) sensitive to radiation in the infrared spectrum reflected from the textile material and configured to generate a corresponding second detector signal, the light sources (2, 3) and detectors (4, 5) being arranged to illuminate and view the same section of the textile material, and a signal processing section (7) configured to

- determine a weighted difference of the two detector signals, that is, of a sample value Sv of a visible light detector (4) and a sample value Si of an infrared light detector (5), by determining a deviation of the ratio Sv/Si of the two detector signals from a predetermined reference ratio Y, and to indicate the presence of a probable contaminant when the deviation exceeds a threshold value;

**characterised in that** the signal processing section (7) is configured to, if the deviation exceeds the threshold value, use of the following quantities for deciding whether a contaminant is present and should be eliminated:

- a deviation Dv of the visible light value Sv from a predetermined reference value Yv;
- a deviation Di of the infrared light value Si from a predetermined reference value Yi;
- a ratio Dv/Di of the two preceding deviations;

wherein the ratio Dv/Di is compared with a predetermined spectral behaviour of different materials for categorising the contaminants;
wherein if the absolute value of the deviation Dv exceeds a predetermined second threshold value, then the presence of certain types of contaminants is confirmed; and
if the absolute value of the deviation Di exceeds a predetermined third threshold value, then the presence of other types of contaminants is confirmed.

2. The apparatus of claim 1, configured to normalise the weighted difference with respect to the second detector signal.

3. The apparatus of claim 2, wherein the signal processing section (7) is configured to determine the weighted difference Dw as

$$Dw = ( (k * Sv) - Si ) / Si$$

where Sv is the value of the first detector signal, and Si is the value of the second detector signal, and k is a substantially constant weighting coefficient.

4. The apparatus of one of claims 1 to 3, wherein the first and second light source (2, 3) are identical, that is, form a combined source that emits both the visible and the infrared light used for the measurements.

5. The apparatus of one of the preceding claims, with the textile material being a yarn (1) and the apparatus further comprising at least a third detector (24) as position detector arranged, with respect to the yarn (1), in opposition to the corresponding light source (2, 3), and wherein the signal processing section (7) is configured to use a signal from the third detector (24) as a positional signal in order to indicate the presence of a contaminant.

6. The apparatus of claim 5, wherein the processing section (7) is configured to indicate the presence of a contaminant only if a change in the positional signal does not exceed a predetermined threshold.

7. The apparatus of one of claims 1 through 4, where the apparatus is arranged to detect foreign substances (12) in a flow of pieces (11) of textile material, in particular in a flow of chunks of cotton (11).

8. The apparatus of one of the preceding claims, configured to adapt the weighting coefficient k in accordance with a long-term average of the first and second detector signal.

9. A method for detecting foreign substances in a textile material, in particular a strand-like textile material or in a flow of pieces of textile material (11), comprising the steps of

• emitting visible light directed at a section of a the textile material,
• emitting radiation in the infrared spectrum directed at the same section of the textile material,
• detecting visible light reflected from the textile material and generating a corresponding first detector signal,
• detecting radiation in reflected from the textile material in the infrared spectrum and generating a corresponding second detector signal,
• determining a weighted difference of the two detector signals, that is, of a sample value Sv of a visible light detector (4) and a sample value Si of an infrared light detector (5), by determining a deviation of the ratio Sv/Si of the two detector signals from a predetermined reference ratio Y, and indicating the presence of a probable contaminant when the deviation exceeds a threshold value;
• **characterised in that** the method comprises the further step of, if the deviation exceeds the threshold value, using the following quantities for deciding whether a contaminant is present and should be eliminated:

◦ a deviation Dv of the visible light value Sv from a predetermined reference value Yv;
◦ a deviation Di of the infrared light value Si from a predetermined reference value Yi;
◦ a ratio Dv/Di of the two preceding deviations;

wherein the ratio Dv/Di is compared with a predetermined spectral behaviour of different materials for categorising the contaminants;
wherein if the absolute value of the deviation Dv exceeds a predetermined second threshold value, then the presence of certain types of contaminants is confirmed; and
if the absolute value of the deviation Di exceeds a predetermined third threshold value, then the presence of other types of contaminants is confirmed.

10. The method of claim 9, comprising the step of

• normalising the weighted difference with respect to the second detector signal, wherein the normalisation is done by determining the weighted difference Dw as

$$Dw = (\,(k * Sv) - Si\,) / Si$$

where Sv is the value of the first detector signal, and Si is the value of the second detector signal, and k is a substantially constant weighting coefficient.

11. The method of one of the claims 9 to 10, comprising the step of

• using a position signal from a third detector (24) in order to indicate the presence of a contaminant in a yarn (1), with the third detector (24) being arranged, with respect to the yarn (1), in opposition to the light sources (2, 3).

12. The method of one of claims 9 through 11, used for detecting foreign substances (12) in a flow of pieces (11) of textile material, in particular in a flow of chunks of cotton (11).

**Patentansprüche**

1. Vorrichtung zum Erkennen von Fremdstoffen in einem Textilmaterial insbesondere in einem strangförmigen Material wie zum Beispiel einem Garn (1) oder in einem Strom von Textilmaterialteilen (11), wobei die Vorrichtung umfasst: eine erste Lichtquelle (2) zum Abstrahlen eines sichtbaren Lichts; eine zweite Lichtquelle (3) zum Abstrahlen einer Strahlung im Infrarotspektrum; einen ersten Detektor (4), der empfindlich für sichtbares Licht ist, das von dem Textilmaterial reflektiert wird, und der konfiguriert ist zum Erzeugen eines entsprechenden ersten Detektorsignals, und einen zweiten Detektor (5), der empfindlich für eine Strahlung im Infrarotspektrum ist, die von dem Textilmaterial reflektiert wird, und der konfiguriert ist zum Erzeugen eines entsprechenden zweiten Detektorsignals, und wobei die Lichtquellen (2, 3) und die Detektoren (4, 5) angeordnet sind, um den gleichen Sektor des Textilmaterials zu beleuchten und zu beobachten; und einen Signalverarbeitungssektor (7), der konfiguriert ist zum

- Ermitteln einer gewichteten Differenz der beiden Detektorsignale, das heißt, eines Probenwerts Sv eines Detektors für sichtbares Licht (4) und eines Probenwerts Si eines Detektors für Infrarotlicht (5), indem eine Abweichung des Verhältnisses Sv/Si der beiden Detektorsignale von einem vorbestimmten Bezugsverhältnis Y ermittelt wird, und zum Anzeigen des Vorhandenseins einer wahrscheinlichen Verunreinigung, wenn die Abweichung einen Schwellenwert überschreitet;

**dadurch gekennzeichnet, dass** der Signalverarbeitungssektor (7) konfiguriert ist zum, wenn die Abweichung den Schwellenwert überschreitet, Verwenden der folgenden Größen zum Entscheiden, ob eine Verunreinigung vorhanden ist und entfernt werden sollte:

• eine Abweichung Dv des sichtbaren Lichtwerts Sv von einem vorbestimmten Bezugswert Yv;
• eine Abweichung Di des Infrarotlichtwerts Si von einem vorbestimmten Bezugswert Yi;
• ein Verhältnis Dv/Di der beiden vorangehenden Abweichungen;

wobei das Verhältnis Dv/Di mit einem vorbestimmten spektralen Verhalten verschiedener Materialien zum Kategorisieren der Verunreinigungen verglichen wird;
wobei, wenn der Absolutwert der Abweichung Dv einen vorbestimmten zweiten Schwellenwert überschreitet, das Vorhandensein von bestimmten Typen von Verunreinigungen bestätigt wird; und
wenn der Absolutwert der Abweichung Di einen vorbestimmten dritten Schwellenwert überschreitet, das Vorhandensein von anderen Typen von Verunreinigungen bestätigt wird.

2. Vorrichtung nach Anspruch 1, die konfiguriert ist zum Normalisieren der gewichteten Differenz in Bezug auf das zweite Detektorsignal.

3. Vorrichtung nach Anspruch 2, wobei der Signalverarbeitungssektor (7) konfiguriert ist zum Ermitteln der gewichteten Differenz Dw als

$$Dw = ((k * Sv) - Si)/Si$$

wobei Sv der Wert des ersten Detektorsignals ist, und Si der Wert des zweiten Detektorsignals ist, und k ein im Wesentlichen konstanter Gewichtungskoeffizient ist.

4. Vorrichtung nach einem der Ansprüche 1 bis 3, wobei die erste und die zweite Lichtquelle (2, 3) identisch sind, das heißt, eine kombinierte Quelle bilden, die sowohl das sichtbare Licht als auch das Infrarotlicht abstrahlt, das für die Messungen verwendet wird.

5. Vorrichtung nach einem der vorhergehenden Ansprüche, wobei das Textilmaterial ein Garn (1) ist und wobei die Vorrichtung außerdem mindestens einen dritten Detektor (24) als einen Positionsmelder umfasst, der in Bezug auf das Garn (1) entgegengesetzt zu der entsprechenden Lichtquelle (2, 3) angeordnet ist, und wobei der Signalverarbeitungssektor (7) konfiguriert ist zum Verwenden eines Signals von dem dritten Detektor (24) als ein Positionssignal, um das Vorhandensein einer Verunreinigung anzuzeigen.

6. Vorrichtung nach Anspruch 5, wobei der Verarbeitungssektor (7) konfiguriert ist zum Anzeigen des Vorhandenseins einer Verunreinigung nur, wenn eine Änderung des Positionssignals einen vorbestimmten Schwellenwert nicht überschreitet.

7. Vorrichtung nach einem der Ansprüche 1 bis 4, wobei die Vorrichtung geeignet ist, Fremdstoffe (12) in einem Strom von Textilmaterialteilen (11) insbesondere in einem Strom von Baumwollballen (11) zu erkennen.

8. Vorrichtung nach einem der vorhergehenden Ansprüche, die konfiguriert ist zum Anpassen des Gewichtungskoeffizienten k gemäß einem Langzeitdurchschnitt des ersten und des zweiten Detektorsignals.

9. Verfahren zum Erkennen von Fremdstoffen in einem Textilmaterial insbesondere in einem strangförmigen Textilmaterial oder in einem Strom von Textilmaterialteilen (11), wobei das Verfahren die folgenden Schritte umfasst:

• Abstrahlen eines sichtbaren Lichts, das auf einen Sektor des Textilmaterials gerichtet ist,
• Abstrahlen einer Strahlung im Infrarotspektrum, das auf den gleichen Sektor des Textilmaterials gerichtet ist,
• Erkennen des sichtbaren Lichts, das von dem Textilmaterial reflektiert wird, und Erzeugen eines entsprechenden ersten Detektorsignals,

• Erkennen der Strahlung in dem Infrarotspektrum, die von dem Textilmaterial reflektiert wird, und Erzeugen eines entsprechenden zweiten Detektorsignals,

• Ermitteln einer gewichteten Differenz der beiden Detektorsignale, das heißt, eines Probenwerts Sv eines Detektors für sichtbares Licht (4) und eines Probenwerts Si eines Detektors für Infrarotlicht (5), indem eine Abweichung des Verhältnisses Sv/Si der beiden Detektorsignale von einem vorbestimmten Bezugsverhältnis Y ermittelt wird, und Anzeigen des Vorhandenseins einer wahrscheinlichen Verunreinigung, wenn die Abweichung einen Schwellenwert überschreitet;

• **dadurch gekennzeichnet, dass** das Verfahren außerdem den folgenden weiteren Schritt umfasst: wenn die Abweichung den Schwellenwert überschreitet, Verwenden der folgenden Größen zum Entscheiden, ob eine Verunreinigung vorhanden ist und entfernt werden sollte:

  ◦ eine Abweichung Dv des sichtbaren Lichtwerts Sv von einem vorbestimmten Bezugswert Yv;
  ◦ eine Abweichung Di des Infrarotlichtwerts Si von einem vorbestimmten Bezugswert Yi;
  ◦ ein Verhältnis Dv/Di der beiden vorangehenden Abweichungen;

wobei das Verhältnis Dv/Di mit einem vorbestimmten spektralen Verhalten verschiedener Materialien zum Kategorisieren der Verunreinigungen verglichen wird;

wobei, wenn der Absolutwert der Abweichung Dv einen vorbestimmten zweiten Schwellenwert überschreitet, das Vorhandensein von bestimmten Typen von Verunreinigungen bestätigt wird; und

wenn der Absolutwert der Abweichung Di einen vorbestimmten dritten Schwellenwert überschreitet, das Vorhandensein von anderen Typen von Verunreinigungen bestätigt wird.

**10.** Verfahren nach Anspruch 9, das den folgenden Schritt umfasst:

• Normalisieren der gewichteten Differenz in Bezug auf das zweite Detektorsignal, wobei das Normalisieren erfolgt, indem die gewichtete Differenz Dw ermittelt wird als:

$$Dw = ((k * Sv) - Si)/Si$$

wobei Sv der Wert des ersten Detektorsignals ist, und Si der Wert des zweiten Detektorsignals ist, und k ein im Wesentlichen konstanter Gewichtungskoeffizient ist.

**11.** Verfahren nach einem der Ansprüche 9 bis 10, das den folgenden Schritt umfasst:

• Verwenden eines Positionssignals von einem dritten Detektor (24), um das Vorhandensein einer Verunreinigung in einem Garn (1) anzuzeigen, wobei der dritte Detektor (24) in Bezug auf das Garn (1) entgegengesetzt zu den Lichtquellen (2, 3) angebracht ist.

**12.** Verfahren nach einem der Ansprüche 9 bis 11, das verwendet wird zum Erkennen von Fremdstoffen (12) in einem Strom von Textilmaterialteilen (11) insbesondere in einem Strom von Baumwollballen (11).

**Revendications**

**1.** Appareil destiné à détecter des substances étrangères dans un matériau textile, en particulier un matériau filiforme tel qu'un fil (1), ou un écoulement de morceaux de matériau textile (11), comprenant une première source de lumière (2) destinée à émettre de la lumière visible, une deuxième source de lumière (3) destinée à émettre un rayonnement dans le spectre infrarouge, un premier détecteur (4) sensible à la lumière visible réfléchie depuis le matériau textile et configuré pour générer un premier signal de détecteur correspondant, et un deuxième détecteur (5) sensible au rayonnement dans le spectre infrarouge réfléchi depuis le matériau textile et configuré pour générer un deuxième signal de détecteur correspondant, les sources de lumière (2, 3) et les détecteurs (4, 5) étant agencés pour éclairer et visualiser la même section du matériau textile, et une section de traitement du signal (7) configurée pour

• déterminer une différence pondérée des deux signaux de détecteur, autrement dit d'une valeur échantillonnée Sv d'un détecteur de lumière visible (4) et d'une valeur échantillonnée Si d'un détecteur de rayonnement infrarouge (5), en déterminant un écart du rapport Sv/Si des deux signaux de détecteur par rapport à un rapport de référence prédéterminé Y, et indiquer la présence d'un contaminant probable quand l'écart dépasse une valeur seuil ;

**caractérisé en ce que** la section de traitement du signal (7) est configurée pour, si l'écart dépasse la valeur seuil, utiliser les quantités suivantes pour décider si un contaminant est présent et devrait être éliminé :

• un écart Dv de la valeur de lumière visible Sv par rapport à une valeur de référence prédéterminée Yv ;
• un écart Di de la valeur de rayonnement infrarouge Si par rapport à une valeur de référence prédéterminée Yi ;
• un rapport Dv/Di des deux écarts précédents ;

dans lequel le rapport Dv/Di est comparé à un comportement spectral prédéterminé de différents matériaux pour catégoriser les contaminants ;
dans lequel, si la valeur absolue de l'écart Dv dépasse une deuxième valeur seuil prédéterminée, alors la présence de certains types de contaminants est confirmée ; et
si la valeur absolue de l'écart Di dépasse une troisième valeur seuil prédéterminée, alors la présence d'autres types de contaminants est confirmée.

2. Appareil de la revendication 1, configuré pour normaliser la différence pondérée par rapport au deuxième signal de détecteur.

3. Appareil de la revendication 2, dans lequel la section de traitement du signal (7) est configurée pour déterminer la différence pondérée Dw sous la forme

$$Dw = ((k*Sv)-Si)/Si$$

où Sv est la valeur du premier signal de détecteur et Si est la valeur du deuxième signal de détecteur, et k est un coefficient de pondération sensiblement constant.

4. Appareil d'une des revendications 1 à 3, dans lequel la première et la deuxième source de lumière (2, 3) sont identiques, autrement dit forment une source combinée qui émet à la fois la lumière visible et le rayonnement infrarouge utilisés pour les mesures.

5. Appareil d'une des revendications précédentes, le matériau textile étant un fil (1) et l'appareil comprenant en outre au moins un troisième détecteur (24) comme détecteur de position disposé, par rapport au fil (1), en opposition à la source de lumière correspondante (2, 3), et dans lequel la section de traitement du signal (7) est configurée pour utiliser un signal provenant du troisième détecteur (24) comme un signal de position afin d'indiquer la présence d'un contaminant.

6. Appareil de la revendication 5, dans lequel la section de traitement (7) est configurée pour indiquer la présence d'un contaminant uniquement si un changement dans le signal de position ne dépasse pas un seuil prédéterminé.

7. Appareil d'une des revendications 1 à 4, l'appareil étant agencé pour détecter des substances étrangères (12) dans un écoulement de morceaux (11) de matériau textile, en particulier dans un écoulement de bouts de coton (11).

8. Appareil d'une des revendications précédentes, configuré pour adapter le coefficient de pondération k en fonction d'une moyenne à long terme du premier et du deuxième signal de détecteur.

9. Procédé de détection de substances étrangères dans un matériau textile, en particulier un matériau textile filiforme, ou dans un écoulement de morceaux de matériau textile (11), comprenant les étapes suivantes :

• émission de lumière visible dirigée vers une section d'un matériau textile,
• émission d'un rayonnement dans le spectre infrarouge dirigé vers la même section du matériau textile,
• détection de lumière visible réfléchie depuis le matériau textile et génération d'un premier signal de détecteur correspondant,
• détection d'un rayonnement réfléchi depuis le matériau textile dans le spectre infrarouge et génération d'un deuxième signal de détecteur correspondant,
• détermination d'une différence pondérée des deux signaux de détecteur, autrement dit d'une valeur échantillonnée Sv d'un détecteur de lumière visible (4) et d'une valeur échantillonnée Si d'un détecteur de rayonnement infrarouge (5), par détermination d'un écart du rapport Sv/Si des deux signaux de détecteur par rapport à un rapport de référence prédéterminé Y, et indication de la présence d'un contaminant probable quand l'écart dépasse une valeur seuil ;
• **caractérisé en ce que** le procédé comprend, si l'écart dépasse la valeur seuil, l'étape supplémentaire d'utilisation des quantités suivantes pour décider si un contaminant est présent et devrait être éliminé :

    ◦ un écart Dv de la valeur de lumière visible Sv par rapport à une valeur de référence prédéterminée Yv ;
    ◦ un écart Di de la valeur de rayonnement infrarouge Si par rapport à une valeur de référence prédéterminée Yi ;
    ◦ un rapport Dv/Di des deux écarts précédents ;

dans lequel le rapport Dv/Di est comparé à un comportement spectral prédéterminé de différents matériaux pour catégoriser les contaminants ;
dans lequel, si la valeur absolue de l'écart Dv dépasse une deuxième valeur seuil prédéterminée, alors la présence de certains types de contaminants est confirmée ; et
si la valeur absolue de l'écart Di dépasse une troisième valeur seuil prédéterminée, alors la présence d'autres types de contaminants est confirmée.

**10.** Procédé de la revendication 9, comprenant l'étape suivante :

• normalisation de la différence pondérée par rapport au deuxième signal de détecteur, la normalisation étant effectuée par détermination de la différence pondérée Dw sous la forme

$$Dw = ((k*Sv)-Si)/Si$$

où Sv est la valeur du premier signal de détecteur et Si est la valeur du deuxième signal de détecteur, et k est un coefficient de pondération sensiblement constant.

**11.** Procédé d'une des revendications 9 à 10, comprenant l'étape suivante :

• utilisation d'un signal de position provenant d'un troisième détecteur (24) afin d'indiquer la présence d'un contaminant dans un fil (1), le troisième détecteur (24) étant disposé, par rapport au fil (1), en opposition aux sources de lumière (2, 3) .

**12.** Procédé d'une des revendications 9 à 11, utilisé pour détecter des substances étrangères (12) dans un écoulement de morceaux (11) de matériau textile, en particulier dans un écoulement de bouts de coton (11).

Fig. 1

Fig. 2

Fig. 3

Fig. 8

EP 1 907 830 B1

Fig. 4

VIS
IR

I_y

Fig. 6

I_y

Fig. 7

15

**Fig. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6175408 B
- EP 0399945 B1
- WO 03008950 A
- US 5915279 A
- EP 0652432 A1
- WO 9313407 A